# EUROPEAN PATENT SPECIFICATION

(11) **EP 1 257 582 B1**
(45) Date of publication and mention of the grant of the patent: **29.04.2009**
(21) Application number: 01912573.1
(22) Date of filing: 26.02.2001
(51) Int. Cl.: A61K 38/39

(54) **METHOD FOR INHIBITING ANGIOGENESIS USING MOLECULES THAT ENHANCE PLASMIN FORMATION OR PROLONG PLASMIN ACTIVITY**
VERFAHREN ZUR HEMMUNG DER ANGIOGENESE UNTER VERWENDUNG VON MOLEKÜLEN DIE DIE PLASMINBILDUNG STEIGERN ODER DIE PLASMINAKTIVITÄT VERLÄNGERN
INHIBITION DE L'ANGIOGENESE AU MOYEN DE MOLECULES QUI AMELIORENT LA FORMATION DE PLASMINE OU PROLONGENT L'ACTIVITE PLASMINIQUE

(30) Priority: 25.02.2000 EP 00200664
(43) Date of publication of application: 20.11.2002
(73) Proprietor: Crossbeta Biosciences B.V., 3584 CX Utrecht (NL)
(72) Inventor: GEBBINK, Martijn, Frans, Ben, Gerard, NL-3755 HL Eemnes (NL); VOEST, Emile, Eugene, NL-3768 AL Soest (NL)
(74) Representative: Prins, Adrianus Willem
(86) International application number: PCT/NL2001/000155
(87) International publication number: WO 2001/062799

(56) References cited:
- EP-A- 0 589 181
- WO-A-00/04052
- WO-A-90/14102
- US-A- 5 981 697
- GECHTMAN ZEEV ET AL: "Synthetic peptides derived from the sequence around the plasmin cleavage site in vitronectin: Use in mapping the PAI-1 binding site." FEBS (FEDERATION OF EUROPEAN BIOCHEMICAL SOCIETIES) LETTERS, vol. 315, no. 3, 1993, pages 293-297, XP002143617 ISSN: 0014-5793
- KOST C ET AL: "Limited plasmin proteolysis of vitronectin. Characterization of the adhesion protein as morpho-regulatory and angiostatin-binding factor" EUROPEAN JOURNAL OF BIOCHEMISTRY,DE,BERLIN, vol. 236, no. 2, 1 March 1996 (1996-03-01), pages 682-688-688, XP002121364 ISSN: 0014-2956
- MACHOVICH RAYMUND ET AL: "Denatured proteins as cofactors for plasminogen activation." ARCHIVES OF BIOCHEMISTRY AND BIOPHYSICS, vol. 344, no. 2, 1997, pages 343-349, XP002175284 ISSN: 0003-9861
- BARENDSZ-JANSON ANNEMARIE F ET AL: "In vitro tumor angiogenesis assays: Plasminogen lysine binding site 1 inhibits in vitro tumor-induced angiogenesis." JOURNAL OF VASCULAR RESEARCH, vol. 35, no. 2, March 1998 (1998-03), pages 109-114, XP000929510 ISSN: 1018-1172
- MACHOVICH RAYMUND ET AL: "Myosin as cofactor and substrate in fibrinolysis." FEBS LETTERS, vol. 407, no. 1, 1997, pages 93-96, XP002175285 ISSN: 0014-5793
- VOSKUILEN M. ET AL: 'Fibrinogen Lysine Residue A.alpha.157 Plays a Crucial Role in the Fibrin-induced Acceleration of Plasminogen Activation, Catalyzed by Tissue-type Plasminogen Activator' THE JOURNAL OF BIOLOGICAL CHEMISTRY vol. 262, no. 13, 05 May 1987, pages 5944 - 5946
- GATELY S. ET AL: 'The mechanism of cancer-mediated conversion of plasminogen to the angiogenesis inhibitor angiostatin' PROCEEDINGS OF THE NATIONAL ACADEMY OF SCIENCE USA vol. 94, September 1997, pages 19868 - 19872, XP001008748

## Description

The present invention relates to methods and compositions for interfering in angiogenesis in a mammalian, in particular a human. More specifically, the present invention relates to methods and means for preventing or at least inhibiting angiogenesis by providing compounds which enhance or sustain the formation of plasmin.
The generation of new blood vessels, called angiogenesis or neovascularization, is essential for tissue growth and tissue repair (Folkman, 1995a; Folkman, 1996; Ossowski and Reich, 1983)(Regulation of angiogenesis (1997) edited by I.D. Golberg & E.M. Rosen, Birkhäuser Verlag, Basel Switzerland). Under normal physiological conditions, angiogenesis is observed during wound healing, fetal and embryonal development and formation of the corpus luteum, endometrium and placenta. Angiogenesis has also been found to play a role in diseases. Persistent, unwanted angiogenesis occurs in a multiplicity of disease states, including tumor growth, metastasis and diabetic retinopathy. In these disease states, prevention of angiogenesis could avert the damage caused by the invasion of new vessels. Strategies to prevent the development of new vessels in tumors and metastases have been effective in suppressing growth of these tumors (Folkman, 1995b; Voest, 1996). Therapies directed at control of the angiogenic process could lead to the abrogation or mitigation of diseases in which angiogenesis is involved.

Angiogenesis is a highly regulated process. Angiogenesis is initiated by the release of angiogenic stimuli, such as vascular endothelial growth factor (VEGF). Such stimuli act on the endothelial cells, which line the lumen of blood vessels. Upon stimulation the endothelial cells mediate the degradation of the basement membrane, which surround the endothelial cells in normal vessels. Angiogenic stimuli induce the formation of a provisional matrix and induce migration, proliferation and invasion of endothelial cells into tissue to form a new vessel.

The formation of provisional matrix is a hallmark of angiogenesis. Endothelial cells use the provisional matrix as substrate for adhesion, migration and invasion. As such the provisional matrix is also essential for endothelial cell survival, i.e. provisional matrix proteins can protect endothelial cells from undergoing apoptosis (Isik et al., 1998). The provisional matrix is formed by the action of many molecules that also play a prominent role in coagulation and fibrinolysis. As such the formation of a provisional matrix resembles the formation and degradation of a blood clot or hemostatic plug. The formation of the provisional matrix is initiated by the action of tissue factor. Tissue factor is present in the subendothelial matrix on cancer cells (Hu et al., 1994) and induced on the cell surface of stimulated endothelial cells (Zucker et al., 1998). Expression of tissue factor has been linked to the angiogeneic properties of malignant tumors (Ruf and Mueller, 1996). As a result of tissue factor action thrombin is formed, which generates fibrin from fibrinogen. The provisional matrix contains many proteins, including vitronectin, that are produced in the liver and derived from blood. These proteins are recruted from blood, when vessels become permeable upon stimulation with angiogenic stimuli and are temporarily deposited to form part of the provisional matrix.

The provisional matrix is continuously generated and broken down, a process called remodeling, until a new functional vessel has been properly formed. Remodeling of the provisional matrix is strictly regulated by the balanced action of molecules, involved in the generation and in the degradation, of the matrix. The formation of the serine protease plasmin through activation of its zymogen plasminogen is a key step in this process. Plasmin mediates proteolysis of the provisional matrix by cleaving fibrin, called fibrinolysis, as well as other matrix components. In addition plasmin mediates proteolysis indirectly by the activation of metalloproteinases, which in turn degrade other components of the extracellular matrix, including collagen. Given its pivotal role in matrix remodeling the formation of plasmin is tightly controlled by the balance between the action of plasminogen activators, plasminogen activator inhibitors and by inhibitors of plasmin, such as α2-antiplasmin. A shift in this balance, by either increasing the levels or activity of inhibitors or by enhancing the formation of plasmin have been shown to have profound effects on either endothelial cell adhesion, migration, angiogenesis, metastasis or tumor growth.
The present invention now describes methods and means based on proteinaceous molecules that enhance or sustain levels of plasmin near or at the site of unwanted angiogenesis through activation of plasminogen through tissue plasminogen activator.
Efficient activation of plasminogen by tissue plasminogen activator (tPA), a serine protease expressed almost exclusively by stimulated endothelial cells (Mandriota and Pepper, 1997), requires the presence of a cofactor, and fibrin is regarded as the principal fibrinolytic stimulator, but other proteins of the extracellular matrix, such as collagens, may also enhance plasminogen activation (Stack et al., 1990). Binding and activation of plasminogen is mediated by carboxyterminal lysine residues that are generated in fibrin during plasmin digestion (Fleury et al., 1993). Removal of the carboxy-terminal lysine residues by carboxypeptidases abrogates the stimulatory effect of fibrin. Thrombin-activatable fibrinolysis inhibitor (TAFI), also named plasma procarboxypeptidase B or procarboxypeptidase U is a physiological fibrinolysis inhibitor (Nesheim et al., 1997). Activition of TAFI is mediated by thrombin or plasmin and thrombin mediated activation of TAFI is greatly enhanced by thrombomodulin, a cell surface protein made almost exclusively by endothelial cells. Therefore TAFI is believed to be a modulator of the provisional matrix as it occurs during disease associated angiogenesis. In summary, the efficient formation of plasmin is mediated by proteins or protein fragments that contain an important carboxyterminal lysine residue. An important aspect of the present invention is to enhance or sustain the formation of plasmin, resulting in at least decreased amounts of provisional matrix and at least decreased ability of the provisional matrix to support angiogenesis. For example, proteinaceous molecules comprising a lysine and/or arginine residue and/or a functional equivalent thereof, capable of providing enhanced levels of plasmin in a mammalian through tPA mediated plasminogen activation may be used to stimulate the formation of plasmin. Alternatively and/or additionally, inhibitors of carboxypeptidases may be used to prevent the removal of carboxyterminal lysine residues that stimulate plasmin formation. This novel approach applies to a variety of angiogenesis mediated diseases.
Thus the present invention describes a proteinaceous molecule comprising a lysine and/or arginine residue and/or a functional equivalent thereof, capable of providing enhanced levels of plasmin in a mammalian through tPA mediated plasminogen activation. A functional equivalent of such a residue or molecule is a residue that is capable of performing the same function as the original residue, i.e. stimulate directly or indirectly the formation of plasmin. The stimulation is typically tPA dependent, therefore a tPA binding site is preferably present in the proteinaceous molecules described in the invention. A partial β-sheet and/or cross β-sheet may also be typically present in the proteinaceous molecules described in the invention. It is understood that this proteinaceous molecules may form a proteinaceous aggregate. A good starting point for obtaining or designing proteinaceous molecules described in the invention is fibrin as component of the extra cellular matrix, which is a natural activator described in the invention. Functional equivalents thereof can be prepared by people of skill in the art without needing further explanations here. For sake of ease of production it is preferred that the proteinaceous molecules described in the invention are about 15- 35 amino acid residues long. It is preferred that the lysine, arginine, or their functional equivalent is carboxy-terminal or becomes carboxy-terminal *in situ*.
Several anti-angiogenic compounds have been used to prevent angiogenesis. The most common approach to inhibit angiogenesis, metastasis or tumor growth is to competitively inhibit angiogenic proteins or molecules. Examples include molecules that block growth factor mediated induction of angiogenesis, such as neutralizing antibodies to vascular endothelial growth factor (Kuiper et al., 1998). Several compounds have been described that inhibit angiogenesis by inhibiting proteases, such as plasminogen activator, plasmin or metalloproteinases. These compounds include plasminogen activator inhibitor 1, aprotinin, batimastat and marimastat. The mechanism of other anti-angiogenic compounds, including thalidomide, some of which may have unwanted side effects, is less well known.

The present invention discloses the use of a novel strategy to prevent angiogenesis. In the present invention molecules are used that function as cofactor in tPA mediated formation of plasmin from plasminogen. Upon treatment with such cofactors excess plasmin is being generated, resulting in enhanced proteolysis and detachment of cells. The strategy is non-competitive and the process very efficient, because a single molecule can catalyze the formation of many plasmin molecules. In addition, by generating excess plasmin, this novel approach offers, in contrast to conventional strategies aimed at inhibiting proteases, a unique, efficient way to destroy the microenvironment and shrink affected pathological tissue. Finally, the strategy is specific for cells that express tPA, i.e. activated endothelial cells.
One of the most prominent applications of the present invention lies in the treatment of cancer, in which degradation of a provisional matrix plays an essential role.
The use of the proteinaceous molecules according to the invention is thus apparent and also part of the present invention.
The invention provides the use of a peptide, comprising fibrin degradation products (FDP) and/or an FDP derived synthetic peptide, which is at least *in-vitro* a cofactor of tPA comprising a lysine and/or arginine residue at or near a carboxy-terminus, further comprising a β-sheet, a cross β-sheet and/or a tPA binding site, capable of providing enhanced levels of plasmin in a mammalian through tPA mediated plasminogen activation for the preparation of a medicament to suppress tumor growth and/or to regress established tumors. Angiogenesis is a prerequisite for the growth and progression of solid tumors. The present invention describes the means for preventing or at least inhibiting angiogenesis by providing compounds which enhance or sustain the formation of plasmin, at or near the site of unwanted angiogenesis (for example invasive tumors with a high grade of neovascularization) through activation of plasminogen through tissue plasminogen activator.
According to the invention it is also possible to provide the presence of molecules according to the invention in situ, by inhibiting their removal, e.g. through the presence of carboxypeptidase inhibitors. The combination of both is especially advantageous.

The invention further provides use of a proteinaceous molecule according to the invention wherein said proteinaceous molecule is derived from a component of the extracellular matrix. Plasmin mediates proteolysis indirectly by the activation of metalloproteinases, which in turn can degrade other components of the extracellular matrix, including collagen. The invention further provides use of a proteinaceous molecule according to the invention, wherein said proteinaceous molecule is derivable by proteolytic cleavage from said extracellular matrix component. The invention provides use of a proteinaceous molecule according to the invention, wherein said lysine, arginine is a residue at or near a carboxy-terminus of said proteinaceous molecule. Preferably said proteinaceous molecule comprises from 15-30 amino acid residues.
The invention provides use of a proteinaceous molecule according to the invention, wherein said proteinaceous molecule comprises a β-sheet, a cross β-sheet, and/or a tPA binding site. Preferably said proteinaceous molecule is denatured. Preferably said proteinaceous molecule is derived from degradation products of extracellular matrix components or protein aggregate degradation products (fibrin).
It is clear to a person skilled in the art that only the essential part or parts of a protein are required in the molecules of the invention. Thus deletions/insertions or mutations in non-relevant parts of the protein are anticipated to be equally or more effective as the entire molecule.

It is also clear to a person skilled in the art that the protein molecule of the invention may contain further functional units derived from different molecules existing in nature or artificial to broaden the functionality of the molecule of invention.
It is also clear to a person skilled in the art that a molecule artificially made, structurally related to the molecule of the invention but not containing a protein moiety are anticipated to be equally or more effective.
The invention also describes a method for the treatment of diseases associated with and/or dependent on angiogenesis comprising administering to a patient an effective amount of a proteinaceous molecule according to the invention capable of providing enhanced levels of plasmin in a mammalian through tPA mediated plasminogen activation, also optionally together with a carboxypeptidase inhibitor. Optionally, both activities are present in one (fusion) molecule.

One of the most frequent angiogeneic disease of childhood is the hemangioma. In most cases, the tumors are benign and regress without intervention. In more severe cases, the tumors progress to large cavernous and infiltrative forms and create clinical complications. Systemic forms of hemangiomas, the hemangiomatoses, have a high mortality rate. Therapy-resistant hemangiomas exist. Angiogenesis is also responsible for damage found in hereditary hemorrhagic telangiectasia. This is an inherited disease characterized by multiple small angiomas, tumors of blood or lymph vessels. The angiomas are found in the skin and mucous membranes, often accompanied by epistaxix (nosebleeds) or gastrointestinal bleeding and sometimes with pulmonary or hepatic arteriovenous fistula.

Angiogenesis is prominent in solid tumor formation and metastasis. Angiogenic factors have been found associated with several solid tumors. Tumors in which angiogenesis is involved include, but are not limited to, solid tumors and benign tumors, such as neurofibroma, trachoma and pyogenic granulomas. Prevention of angiogenesis could halt the growth of these tumors and the resultant damage.

Angiogenesis has been associated with blood-born tumors, such as leukemias, any of the various acute or chronic neoplastic diseases of the bone marrow in which unrestrained proliferation of white blood cells occurs, usually accompanied by anemia, impaired blood clotting, and enlargement of the lymph nodes, liver and spleen. It is believed that angiogenesis plays a role in the abnormalities in the bone marrow that give rise to leukemia-like tumors.

Angiogenesis is important in tumor metastasis. Angiogenesis allows tumor cells to enter the blood stream and to settle into a secondary site. Therefore, prevention of angiogenesis could lead to the prevention of metastasis.

The present invention describes among other things a method for the treatment of angiogenesis dependent or related diseases by using molecules that efficiently promote or sustain tPA mediated plasmin formation. A major advantage for therapeutical application is that the method is selective for tPA. This implies that molecules based on the invention have a restricted and localized action, namely at sites were tPA is available, such as on the surface of activated endothelial cells or certain tumor cells. In addition, the method offers the advantage that through enhanced plasmin formation, rather than decreased plasmin formation, the pathological tissue may decline.
The invention further describes a method for the treatment of a disease according to the invention further comprising administering a carboxypeptidase inhibitor. The invention further provides use of a proteinaceous molecule according to the invention in the preparation of a pharmaceutical formulation. Suitable pharmaceutical formulations are known and they may be in dosage forms such as tablets, pills, powders, suspensions, capsules, suppositories, injection preparations, ointments, eye drops etc.

The invention provides a pharmaceutical formulation according to the invention further comprising a carboxypeptidase inhibitor.

Upon further study of the specification, drawings and appended claims, objects and advantages of this invention will become apparent to those skilled in the art.

### EXAMPLES

### EXAMPLE 1 : Effect of fibrin-degradation products (FDP) on subcutaneous tumor growth.

A model of subcutaneous tumor growth of a mouse C26 colon carcinoma was used to evaluate the effects of FDP. Eleven days after tumor cell inoculation tumors of the control group had reached a volume of 2719 ± 366 mm³. In mice treated with FDP the mean tumor growth was 719 ± 188 mm³. At similar concentrations, endostatin, another molecule with the ability to stimulate tPA mediated plasmin formation (see below), suppressed tumor growth as well but was slightly less effectively (tumor volume 1112 ± *372 mm³*). Treatment with tPA, which generates plasmin, suppressed tumor growth to a similar degree (tumor volume 492 ± 215 mm³) as FDP. These result demonstrate that two molecules which fullfil the criteria of the proposed invention potently inhibited tumor growth.

### EXAMPLE 2 : Effect of FDP on endothelial cells in vitro.

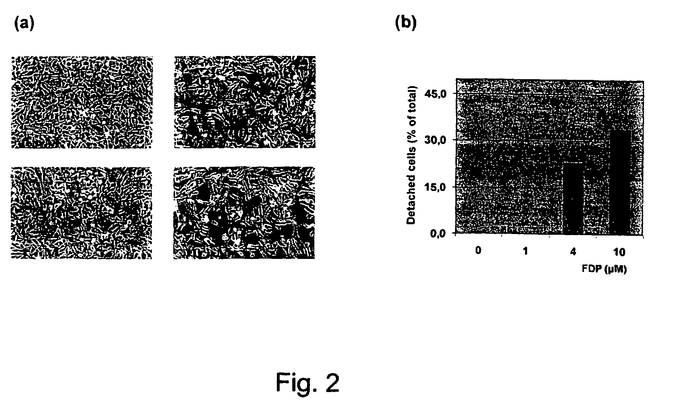

Treatment of BPAEC cells grown in monolayers caused dramatic morphological changes within 24 hr, while untreated cells retain their characteristic cell shape (Fig 2a). More than 30% of the cells treated with FDP detached from the substratum (Fig 2b).

EXAMPLE 3 : Effect of FDP, FPD derived synthetic peptide (aa 148-160), fragment (aa. 262-367) vitronectin and endostatin on plasmin generation. Various concentrations were added.

### Materials and Methods

### Endostatin

The cDNA for murine endostatin (kindly provided by dr. Fukai, Boston) was amplified by PCR and cloned into the prokaryotic expression vector pET15b.

Recombinant murine endostatin was produced by *Escherichia coli* and purified on Ni²⁺-NTA-beads (Qiagen) as described (Boehm et al., 1997; O'Reilly et al., 1997).

### Fibrin Degradation Products (FDP)

Fibrin degradation products (FDP) were generated by plasmin digestion of fibrin. Human fibrinogen (Sigma, The Netherlands) at a concentration of 5 mg/ml was allowed to clot in 25 mM Tris-HCl pH 7.4, 150 mM NaCl by the addition of thrombin (1.32 µM final concentration) for 3 hr at 37 °C (thrombin was a generous gift of Dr. W. Kisiel, University of New Mexico, Albequerque, NM, USA). Clot lysis was accomplished by addition of plasmin (Roche, ). Plasmin was added at a molar ratio (plasmin:fibrinogen) of 1:300. Lysis was performed for 20 hr at room temperature. After centrifugation the supernatant was passed through an aprotinin-sepharose column to remove plasmin. FDP were stored at -20 °C. Activity was determined using a plasminogen activation assay.

### Measurement of plasmin activity

Reactions were performed at 37 °C in HBS buffer (20 mM Hepes, 4 mM KCl, 137 mM NaCl, 3 mM CaCl₂, 0.1 % BSA, pH 7.4) containing 50 µg/ml plasminogen with or without the addition of endostatin or FDP. The reactions were started by the addition tPA at a final concentration of 30 U/ml. At several time points, 20 µl samples were taken and stopped with 20 µl buffer containing 150 mM εACA and 150 mM EDTA. Plasmin activity was determined in 96-well plates after the addition of 20 µl chromogenic substrate S-2251 at a final concentration of 1.6 mM. Increase in absorbance was measured at 405 nm for 10 min.

### Cells and culture conditions

The mouse colon adenocarcinoma cell line C-26 was maintained as a monolayer culture in Dulbecco's Minimal Essential Medium (DMEM) supplemented with 10% heat-inactivated fetal calf serum, penicillin (100 units/ml), and streptomycin (100 µg/ml) in a 5% CO₂ environment. Confluent cultures were harvested by brief trypsinization (0.05 trypsin in 0.02% EDTA), washed 3 times with PBS, and resuspended to a final concentration of 5x10⁶ cells/ml. The presence of single cell suspension was confirmed by phase contrast microscopy, and cell viability was determined by trypan blue staining.

### Cell detachment assay

BPAEC (CCL-209) was obtained from the American Type Culture Collection (Rockville, MD) BPAEC were grown in Dulbecco's Modified Essential Medium (DMEM) with 20% fetal calf serum (FCS) and antibodies. BPAEC were seeded in 24-well culture dishes and solvent control in DMEM containing 20 mM Hepes and 10 % FCS. Cell morphologh was examined by phase contrast microscopy. After 48 to 72 hours the detached cells were removed and the remaining attached cells were removed by trypsin exposure and counted. The percentage of detached cells was calculated.

### Mice

Male BALB/c mice were purchased from the General Animal Laboratory, University Medical Centre (Utrecht, The Netherlands). Animals were maintained with food and water ad libitum and kept on a 12-hour light/12 hour dark cycle. All animal studies were conducted on male, 6 to 8 week old mice.

### Tumor experiments

Male BALB/c mice were inoculated with 10⁶ C26 colon carcinoma cells. Mice were treated daily by subcutanous injection with either saline or the compound to be tested for a period of approximately 14 days. Serial caliper measurements of perpendicular diameters were used to calculate tumor volumes in mm³ using the formula: longest diameter x shortest diameter² x 0.52.

### Statistical Analysis

The statistical significance of differences between groups was calculated by applying Students 2-tailed t-test. Results are presented as the mean ± standard error of the mean.

For illustrative purposes only:
Denatured Anti-thrombin III stimulates tPA mediated plasmin formation

### Anti-thrombin (ATIII) assay

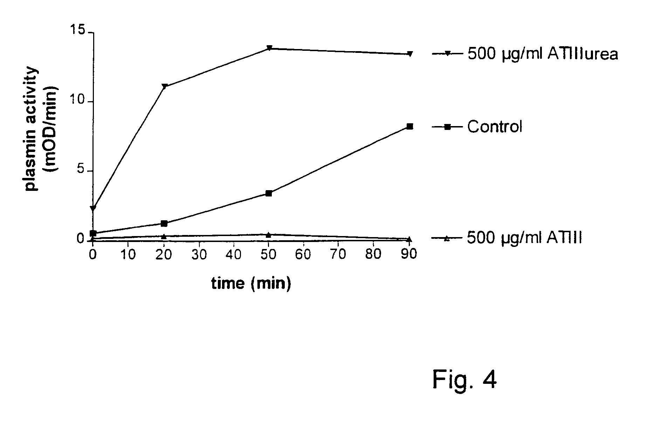

Active anti-thrombin (ATIII) was purified by the method of De Swart (Bonno Bouma) et al., (1984). Denatured ATIII was made by dialysis of active ATIII against 8 M urea (O/N). Urea was removed after extensive dialysis against 20 mM Tris, 150 mM NaCl, pH 7.4. 500 µg/ml of active or denatured ATIII was added to HBS (137 mM NaCl, 4 mM KCl, 3 mM CaCl₂, 20 mM Hepes pH 7.4) containing 100 µg/ml plasminogen. Plasmin formation was initiated by addition of 50 U/ml tPA at 37°C and measured using S2251 (Fig 4).

### Figure Legend

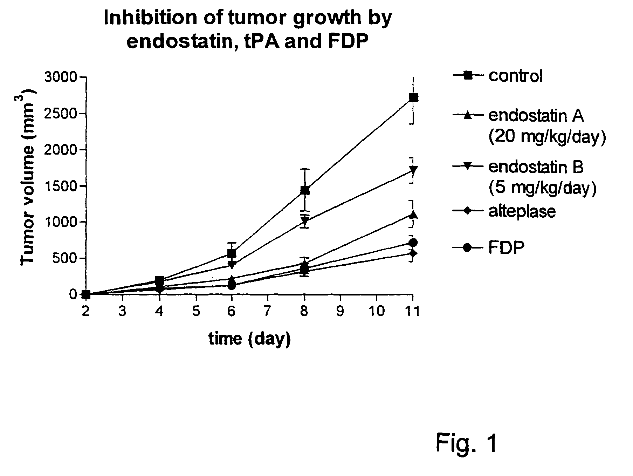

Figure 1 : Effect of FDP on tumor growth. For comparison, treatments with Alteplase® (recombinant tPA) or endostatin is shown. FDP was given continuously using Alzet® pumps (Alza, Palo Alto, CA, USA, type 2001 or 2002) loaded with 200 µl FDP (5 mg/ml). In addition every other day FDP was given subcutaneously at a dose of 7 mg/kg.
Figure 2 : Effect of FDP on endothelial cell attachment. (a) Micrographs showing BPAEC monolayers of (I) control cells treated with PBS for 24 hr; (II) cells treated with 1 µM FDP; (III) cells treated with 4 µM FDP; cells treated with 10 µM FDP.

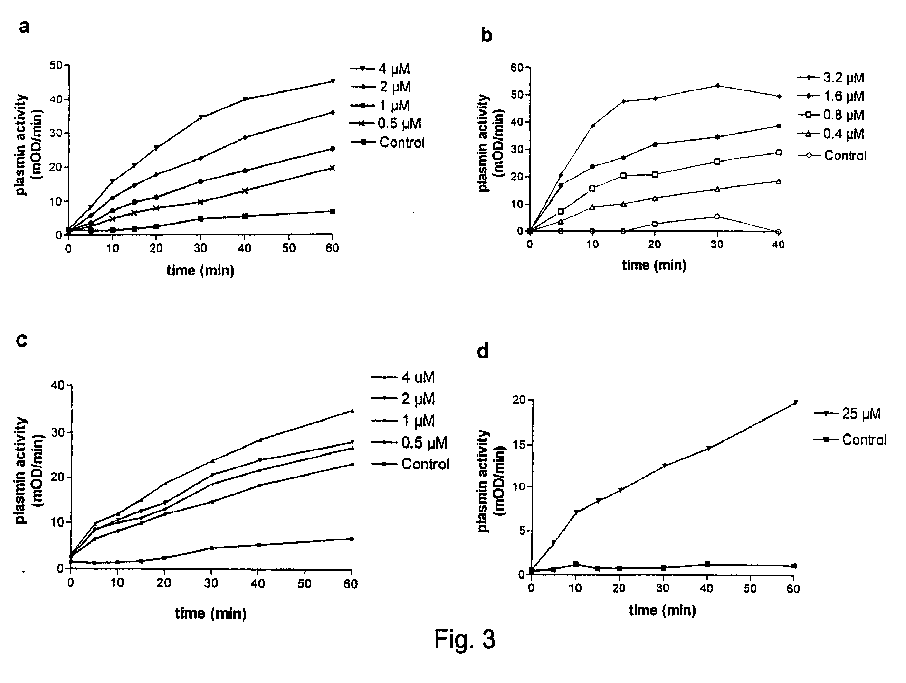

Figure 3 : Effect on plasmin activity of endostatin (a), vitronectin fragment + (aa. 262-367) (b) FDP (c), peptide derived from FDP (aa. 148-160) (d).
Figure 4. Denatured Anti-thrombin III stimulates tPA mediated plasmin formation (for illustrative purposes only).

### Reference List

Boehm,T., Folkman,J., Browder,T., and O'Reilly,M.S. (1997). Antiangiogenic therapy of experimental cancer does not induce acquired drug resistance. Nature 390, 404-407.
Fleury,V., Loyau,S., Lijnen,H.R., Nieuwenhuizen,W., and Angles-Cano,E. (1993). Molecular assembly of plasminogen and tissue-type plasminogen activator on an evolving fibrin surface. Eur. J. Biochem. 216, 549-556.
Folkman,J. (1995a). Angiogenesis in cancer, vascular, rheumatoid and other disease. Nat. Med. 1, 27-31.
Folkman,J. (1995b). Clinical applications of research on angiogenesis. Semin. Med. Beth Israel Hosp. 333, 1757-1763.
Folkman,J. (1996). Fighting cancer by attacking its blood supply. Sci. Am. 275, 150-154.
Ge,M., Tang,G., Ryan,T.J., and Malik,A.B. (1992). Fibrinogen degradation product fragment D induces endothelial cell detachment by activation of cell-mediated fibrinolysis. J. Clin. Invest 90, 2508-2516.
Hu,T., Bach,R.R., Horton,R., Konigsberg,W.H., and Todd,M.B. (1994). Procoagulant activity in cancer cells is dependent on tissue factor expression. Oncol. Res 6, 321-327.
Isik,F.F., Gibran,N.S., Jang,Y.C., Sandell,L., and Schwartz,S.M. (1998). Vitronectin decreases microvascular endothelial cell apoptosis. J Cell Physiol. 175, 149-155.
Kuiper, R.A., Schellekens, J.H., Blijham, G.H. , Beyeren, J.H. and Voest, E.E. (1998). Clinical research on antianjiogenic therapy. Pharmacol. REs. 37(1), 1-16.
Mandriota,S.J. and Pepper,M.S. (1997). Vascular endothelial growth factor-induced in vitro angiogenesis and plasminogen activator expression are dependent on endogenous basic fibroblast growth factor. J. Cell Sci. 110, 2293-2302.
Nesheim,M., Wang,W., Boffa, M., Nagashima,M., Morser,J., and Bajzar,L. (1997). Thrombin, thrombomodulin and TAFI in the molecular link between coagulation and fibrinolysis. Thromb. Haemost. 78, 386-391.
O'Reilly,M.S., Boehm,T., Shing,Y., Fukai,N., Vasios,G., Lane,W.S., Flynn,E., Birkhead,J.R., Olsen,B.R. , and Folkman,J. (1997). Endostatin: an endogenous inhibitor of angiogenesis and tumor growth. Cell 88, 277-285.
Ossowski,L.K. and Reich,E. (1983). Antibodies to plasminogen activator inhibit human tumor metastasis. Cell 35, 611-619.
Ruf,W. and Mueller,B.M. (1996). Tissue factor in cancer angiogenesis and metastasis. Curr. Opin. Hematol. 3, 379-384.
Stack,S., Gonzalez-Gronow,M., and Pizzo,S.V. (1990). Regulation of plasminogen activation by components of the extracellular matrix. Biochemistry 29, 4966-4970.
Voest,E.E. (1996). Inhibitors of angiogenesis in a clinical perspective. Anticancer Drugs 7, 727.
Voskuilen, M., Vermond, A., Veeneman G.H., van Boom J.H., Klasen E.A., Zegers, N.D., and Nieuwenhuizen, W. (1987). Fibrinogen Lysine residue Ax 157 plays a crucial role in the fibrin-induced acceleration of plasminogen activation, catalyzed by tissue-type plasminogen activator, JBC 262, 5944-5946.
Zucker,S., Mirza,H., Conner,C.E., Lorenz,A.F., Drews,M.H., Bahou,W.F., and Jesty,J. (1998). Vascular endothelial growth factor induces tissue factor and matrix metalloproteinase production in endothelial cells: conversion of prothrombin to thrombin results in progelatinase A activation and cell proliferation. Int. J Cancer 75, 780-786

## Claims

1. Use of a peptide, comprising fibrin degradation products (FDP) and/or an FDP derived synthetic peptide, which is at least *in*-*vitro* a cofactor of tPA, comprising a lysine and/or arginine residue at or near a carboxy-terminus, further comprising a 6-sheet, a cross 6-sheet and/or a tPA binding site, capable of providing enhanced levels of plasmin in a mammalian through tPA mediated plasminogen activation for the preparation of a medicament to suppress tumor growth and/or to regress established tumors.

2. Use according to claim 1, wherein the peptide consists of a peptide derived from FDP (aa 148-160).

3. Use of a peptide a cording to any of claims 1 to 2 in the preparation of a medicament for the degradation of a provisional matrix.

4. Use according to any one of claims 1-3, in combination with an inhibitor of carboxypeptidase.

5. Use according to any one of claims 1-4, wherein said peptide is derived from a component of the extracellular matrix.

6. Use according to claim 5, wherein said peptide is derivable by proteolytic cleavage from said extracellular matrix component.

7. Use according to anyone of claims 1-6 wherein said peptide is denatured.

## Patentansprüche

1. Verwendung eines Peptids, das Fibrin-Abbauprodukte (fibrin degradation products = FDP) und/oder ein von FDP abgeleitetes synthetisches Peptid umfasst, das wenigstens in vitro ein Cofaktor von tPA ist, umfassend einen Lysin- und/oder Argininrest an oder nahe einem Carboxyterminus, außerdem umfassend ein β-Faltblatt, ein cross-β-Faltblatt und/oder eine tPA-Bindungsstelle, das fähig ist, erhöhte Konzentrationen an Plasmin in einem Säuger durch tPA-vermittelte Plasminogenaktivierung bereitzustellen, für die Herstellung eines Medikaments, um Tumorwachstum zu supprimieren und/oder etablierte Tumore zurückzubilden.

2. Verwendung gemäß Anspruch 1, wobei das Peptid aus einem Peptid besteht, das von FDP abgeleitet ist (aa 148-160).

3. Verwendung eines Peptids gemäß einem der Ansprüche 1 bis 2 bei der Herstellung eines Medikaments für den Abbau einer provisorischen Matrix.

4. Verwendung gemäß einem der Ansprüche 1 bis 3 in Kombination mit einem Inhibitor von Carboxypeptidase.

5. Verwendung gemäß einem der Ansprüche 1 bis 4, wobei das Peptid von einer Komponente der extrazellulären Matrix abgeleitet ist.

6. Verwendung gemäß Anspruch 5, wobei das Peptid durch proteolytische Spaltung aus der Komponente der extrazellulären Matrix ableitbar ist.

7. Verwendung gemäß einem der Ansprüche 1 bis 6, wobei das Peptid denaturiert ist.

## Revendications

1. Utilisation d'un peptide, comprenant des produits de dégradation de la fibrine (PDF) et/ou un peptide synthétique dérivé de PDF, qui est au moins *in vitro* un cofacteur du tPA comprenant un résidu lysine et/ou arginine au niveau ou au voisinage d'une terminaison carboxy, comprenant en outre un feuillet β, un feuillet β antiparallèle et/ou un site de liaison au tPA et permettant d'obtenir des taux améliorés de plasmine chez un mammifère par activation du plasminogène médiée par le tPA, pour la préparation d'un médicament destiné à supprimer la croissance tumorale et/ou à faire régresser des tumeurs établies.

2. Utilisation selon la revendication 1, dans laquelle le peptide est constitué d'un peptide dérivé de PDF (acides aminés 148 à 160).

3. Utilisation d'un peptide selon l'une quelconque des revendications 1 à 2 pour préparer un médicament destiné à dégrader une matrice provisoire.

4. Utilisation selon l'une quelconque des revendications 1 à 3, en combinaison avec un inhibiteur de carboxypeptidase.

5. Utilisation selon l'une quelconque des revendications 1 à 4, dans laquelle ledit peptide est dérivé d'un composant de la matrice extracellulaire.

6. Utilisation selon la revendication 5, dans laquelle ledit peptide peut être dérivé par dissociation protéolytique dudit composant de la matrice extracellulaire.

7. Utilisation selon l'une quelconque des revendications 1 à 6, dans laquelle ledit peptide est dénaturé.
